# EUROPEAN PATENT APPLICATION

(11) **EP 1 229 128 A1**
(43) Date of publication of application: **07.08.2002**
(21) Application number: 01102106.0
(22) Date of filing: 31.01.2001
(51) Int. Cl.: C12Q 1/68

(54) **New method for genotype determination**

(71) Applicant: Roche Diagnostics GmbH, 68305 Mannheim (DE); F. Hoffmann-La Roche AG, 4070 Basel (CH)
(72) Inventor: Costa, Jean-Marc, 75014 Paris (FR)

(57) **Abstract**

The present invention is directed to a new method for genotype determination at a specific gene locus of an individual or a fetus comprising (i) amplifying a first sequence of said gene locus and a second sequence of a second reference gene locus from DNA originating from a sample containing biological material of said individual or fetus (ii) Monitoring both amplifications preferably in real time and determining the amount of amplification products after each cycle, and (iii) Calculating the ratio between the amount of DNA from the first gene locus and the amount of DNA from the second gene locus. The new method is useful for a variety of applications, especially for detection of chromosomal abnormalities in fetal cells.

## Description

The present invention relates to the field of genotype determination. More specifically, the new invention relates to the field of determination of genotypes using nucleic acid amplification technologies like the Polymerase Chain Reaction (PCR).

Determination of a certain genotype of an individual sometimes may be a an essential diagnostic tool in order to decide on the medical treatment of a patient, e.g. with regard for his susceptibility for a certain therapeutic drug.

Especially for prenatal diagnostics, genotype determination is performed frequently. For example, in case pregnant women are homozygously negative with respect to the RhD blood group system antigen, it is important to determine the fetal RhD genotype, since RhD-heterozygous babies from RhD-homozygously negative mothers **could** suffer from allo-immunization reactions and hemolytic anemia, in case no prophylactic treatment is taking place (**Whittle, Arch Dis Child 1992 Jan;67**(**1 Spec No**):**65-8**). Another important field is the test for trisomy 21 resulting in Down syndrome, trisomy 18 resulting in Edwards syndrome and trisomy 13 resulting in Patau syndrome, since there exists an increased risk in case the pregnant women are older than 35 years.

In order to perform genotyping on fetal DNA, fetal cells are usually obtained by amniocentesis and subjected to further investigation. Alternatively, few fetal cells may be collected from the mother's blood stream, which, however, usually need to be enriched by immunological or physical methods prior to subsequent genotyping experiments (**Pertl et al, Semin Perinatol 1999 Oct;23(5):393-402**). Traces of free floating fetal DNA has also been observed in the blood stream of the mother (**Lo et al, Lancet. 1997 Aug 16;350(9076):485-7**). However, due to the presence of maternal DNA background, a genotype analysis regarding the fetal DNA is only possible in very specific cases like RhD in the background of a RhD recessive mother (Lo et al., New England J. of Medicine 339, p. 1734-1738, 1998), but not for other cases like e.g. Trisomy 13, 18, or 21.

In the past, genotype determination usually has been performed by means of conventional microscopy, which requires a labour extensive and sophisticated chromosomal spreading. Eventually, these methods have been combined with in situ hybridization protocols like the FISH technique (**Philip et al, Prenat Diagn 1994 Dec;14(13):1203-15**). Alternatively, DNA Southern Blot based molecular methods may now be applied. These include, for example, conventional Restriction Fragment Length Polymorphism analysis. However, due to the limited amount of available sample material, these methods are not applicable for prenatal diagnostics.

Among the number of different analytical methods that detect and quantify nucleic acid sequences, Polymerase Chain Reaction (PCR) has become the most powerful and widespread technology, the principles of which are disclosed in U.S. 4,683,195 and U.S. 4,683,102 (Mullis et al.). However, a typical PCR reaction by itself only yields qualitative data, since, after a phase of exponential or progressive amplification, the amount of amplified nucleic acid reaches a plateau, such that the amount of generated reaction product is not proportional to the initial concentration of the template DNA.

As a consequence, many different PCR based protocols have been developed in order to obtain reliable and reproducible quantitative data. Generally, three different basic principles can be discriminated:
i) Relative Quantification of a target nucleic acid compared to a standard within the sample ( e. g. expression of a housekeeping gene in case of RT-PCR)
ii) Quantification with competitive PCR using an internal standard
iii) Quantification of target DNA using an external standard

A major improvement in the generation of quantitative data derives from the possibility of measuring the kinetics of a PCR reaction by On-Line detection. This has become possible recently by means of detecting the amplicon through fluorescence monitoring. Examples of such techniques are disclosed in detail in WO 97/46707, WO 97/46712 and WO 97/46714 (Wittwer et al.), the disclosures of which are hereby incorporated by reference.

All these methods can easily be designed as assays with a dynamic range of less than about 2 orders of magnitude, i.e. wherein the concentrations of the target nucleic acids in two different samples to be determined do not differ more than a hundred fold. However, regarding the lower discrimination limit, prior to the new invention no reliably protocols have been published, which disclose the discrimination of concentration differences between two target concentrations that only differ from each other by not more than 50%.

Several detection formats based on target nucleic acid dependent fluorescent signaling have been disclosed, which enable continuous monitoring of the generation of amplification products (reviewed in Wittwer et al., Biotechniques, Vol. 22, No, 1, 130-138, 1997). These detection formats include but are not limited to:
**1. Use of fluorescent double-stranded DNA recognizing compounds**
   Since the amount of double stranded amplification product usually exceeds the amount of nucleic acid originally present in the sample to be analyzed, double-stranded DNA specific dyes may be used, which upon excitation with an appropriate wavelength show enhanced fluorescence only if they are bound to double-stranded DNA. Preferably, only those dyes may be used which like SYBR Green I (Molecular Probes), for example, do not affect the efficiency of the PCR reaction.
**2. Increased fluorescence resonance energy transfer upon hybridization**
   For this detection format, two oligonucleotide hybridization probes each labeled with a fluorescent moiety are used which are capable of hybridizing to adjacent but non overlapping regions of one strand of the amplification product. Preferably, one oligonucleotide is labeled at the 5' end and the second oligonucleotide is labeled at the 3' end. When hybridized to the target DNA, the two fluorescent labels are brought into close contact, such that fluorescence resonance energy transfer between the two fluorescent moieties can take place. As a consequence, the hybridization can be monitored through excitation of the donor moiety and subsequent measurement of fluorescence emission of the second acceptor moiety.
   In a similar embodiment, only one fluorescently labeled probe is used, which together with one appropriately labeled primer may also serve as a specific FRET pair (Bernard et al., Analytical Biochemistry 235, p. 101-107 (1998)).
**3. Taq Man principle**
   In order to detect the amplification product, a single-stranded hybridization probe is used, which is labeled with a fluorescent entity, the fluorescence emission of which is quenched by a second label on the same probe which may act as a quenching compound. During the annealing step of the PCR reaction, the probe hybridizes to its target sequence, and, subsequently, during the extension of the primer, the DNA polymerase having a 5'-3'-exonuclease activity digests the hybridization probe into smaller pieces, such that the fluorescent entity is separated from the quencher compound. After appropriate excitation, fluorescence emission can be monitored as an indicator of accumulating amplification product.
**4. Molecular beacons**
   Similar to the Taq Man Probes, a molecular beacon oligonucleotide is labeled with a fluorescent compound and a quencher compound, which due to the secondary structure of the molecule are in dose vicinity to each other. Upon binding to the target DNA, the intramolecular hydrogen bonding is broken, and the fluorescent compound located at one end of the probe is separated from the quencher compound, which is located at the opposite end of the probe (Lizardi et al., US 5, 118,801).

All the prior art methods discussed above, which may be used for the assessment of an allelic status, however, show major disadvantages:
- Determination of an allelic status using microscopic techniques requires time consuming chromosomal spreading and staining procedures
- Southern Blot based hybridization methods like Restriction Fragment Length Polymorphism require a large amount of starting material, which especially in prenatal diagnosis is not obtainable.
- Conventional qualitative prior art Nucleic Acid amplification methods like PCR are useful in order to detect the presence or absence of a specific allele, however, these methods are not suited in order to discriminate between a homo- or heterozygous status or detect the existence of low copy allelic amplification.
- Conventional quantitative prior art Nucleic Acid amplification methods require an extensive calibration and can not easily discriminate between one, two or three copies of a target nucleic acid originally present in the sample.

### Summary of the invention

Therefore, there exists a need in the art for a fast and easy method which allows for a rapid determination of an allelic status of a specific gene locus or even a complete chromosome starting from a limited amount of sample material.

Thus, the new invention is directed to a method for genotype determination at a specific gene locus of an individual or a fetus comprising
a) Amplifying a first sequence of said gene locus and a second sequence of a second reference gene locus from DNA originating from a sample containing biological material of said individual or fetus
b) Quantifying the original amount of DNA of said first gene locus and said second gene locus present in the sample
c) Calculating the ratio between the amount of DNA from the first gene locus and the amount of DNA from the second gene locus originally present in the sample prior to the amplification reaction

Thus, the ratio is indicative for the genotype of the sample to be analyzed.

More specifically, the invention is directed to a method for genotype determination at specific gene locus of an individual or a fetus comprising
a) Amplifying a first sequence of said gene locus and a second sequence of a second reference gene locus from DNA originating from a sample containing biological material of said individual or fetus

a) Monitoring both amplifications in real time and determining the amount of amplification products after each cycle
b) Calculating the ratio between the amount of DNA from the first gene locus and the amount of DNA from the second gene locus

In a major aspect of the invention, a sample of fetal cells is used, which has been obtained either from or amniocentesis or from maternal blood. In the latter case, it is preferred if the fetal cells have been isolated by immuno-enrichment or physical enrichment.

In the context of the new method, it has been proven to be advantageous, if the first and the second sequence are amplified and detected in one tube.

Detection of the amplification products is preferably obtained by means of detecting fluorescent signals and most preferably by means of using a couple of FRET/Hybridization probes.

One specific embodiment of the invention is directed to the methods disclosed above, wherein the MBP gene locus, the SOD gene locus or the RhD gene locus are amplified and analyzed.

It is within the scope of the present invention to use the claimed method in order to detect chromosomal abnormalities. Among these abnormalities, chromosomal aberrations like trisomy 21, trisomy 18, trisomy 13 or microdeletional syndromes may be detected according to the invention.

The new invention may also be used to determine a female carrier status for a Chromosome X-linked genetic disorder like for example, Haemophilia or Myopathy.

Moreover, the new method may also be used for sex determination, if DNA from fetal cells is analyzed.

### Detailed description of the invention

In a first and predominant aspect, the present invention provides a general method for genotype determination at a specific gene locus of an individual or a fetus comprising
a) Amplifying a first sequence of said gene locus and a second sequence of a second reference gene locus from DNA originating from a sample containing biological material of said individual or fetus
b) Quantifying the original amount of DNA of said first gene locus and said second gene locus present in the sample
c) Calculating the ratio between the amount of DNA from the first gene locus and the amount of DNA from the second gene locus originally present in the sample prior to the amplification reaction

Amplification of said first and second sequence is usually performed by means of PCR. Quantification of the original amount of DNA can be achieved by any method known in the art such as application of an external standard or application of an internal standard for competitive PCR. In the latter case, the internal standard is usually amplified with the same primers like the target nucleic acid itself. Preferably, the sequence of the internal competitor is similar to the sequence of the target nucleic acid, such that both are amplified with about the same efficiency. It is also within the scope of the invention, in case the amount of target DNA is not determined in an absolute value rather than as a relative value as compared to the external standard or the competitor DNA without knowing the actual concentration of the target- or competitor DNA itself.

In the context of this invention, genotype determination is understood as determination of the gene dosis of a specific allele present in the genome to be analyzed. According to the invention, concentrations of amplification products originating from 2 different gene loci are always compared with each other. As will be shown in the examples, the new method is able to identify differences between one and two copy numbers of an allele (for example in case of of RhD and Factor VIII), or even between two or three copies of an allele (for example in case of trisomy detection). In the latter case, the new invention provides a method in order to discriminate differences in gene dosage of as few as 50%.

The term "individual" in the context of this invention not only comprises an individual human being, but also an individual animal or plant specimen. Furthermore, individual may also mean a particular strain of a microorganism, originating from one single clone.

The present invention is especially directed to a method for genotype determination at a specific gene locus of an individual or a fetus comprising
b) Amplifying a first sequence of said gene locus and a second sequence of a second reference gene locus from DNA originating from a sample containing biological material of said individual or fetus
c) Monitoring both amplifications in real time and determining the amount of amplification products after each cycle
d) Calculating the ratio between the amount of DNA from the first gene locus and the amount of DNA from the second gene locus

In this preferred embodiment, the assay is performed in a homogeneous detection format in Real Time. That means, a suitable hybridization probe is already present during the amplification reaction. The hybridization probe preferably carries a fluorescent label which is detectable after appropriate excitation. Due to the possibility of kinetic measurements during the amplification itself, monitoring the reaction in Real Time strongly facilitates the quantification of the target DNAs. Again all methods and instruments known in the art for Real Time PCR quantification may be used.

In this context, however, it is important to know, that the signals obtained by Real Time PCR do not exclusively reflect the amount of target DNAs present in the sample, since the intensity of the signals is also dependent on the sensitivity of the detection system applied. The sensitivity itself is influenced by several parameters like e.g. melting point of the specific hybridization probe or fluorescent quantum yield. As a consequence, for example, homozygous existence of a first allele compared to heterozygous existence of a second reference allele in this type measurements does not exactly result in a ratio of 2:1. Nevertheless, the method according to the new invention allows for discrimination between a homozygous and a heterozygous state of the same allele (see examples below).

A specific aspect of the invention is directed to a method for genotype determination at a specific gene locus of a fetus comprising
a) Isolating fetal cells preferably by Immuno-enrichment or physical enrichment
b) Amplifying a first sequence of said gene locus and a second sequence of a second reference gene locus from DNA originating from cells isolated in step a))
c) Monitoring both amplifications in real time and determining the amount of amplification products after each cycle
d) Calculating the ratio between the amount of DNA from the first gene locus and the amount of DNA from the second gene locus originally present in the sample prior to the amplification reaction method

In case a prenatal analysis is performed, it is advantageous either to use cells obtained by amniocentesis or, alternatively to enrich fetal cells obtained from maternal blood by immunological methods, preferably with an antibody that binds to a surface antigen of a fetal cell, which is not expressed on the surface of cells found in the maternal blood vessel system (**Wang et al, Cytometry 2000 Mar 1;39(3):224-30**). In a still further alternative approach, fetal cells may be enriched by a Ficoll gradient centrifugation according to the teaching of (**Samura et al, Prenat Diagn 2000 Apr;20(4):281-6**)

One possibility for Immuno-enrichment is the use of a laser based cell sorting system. In this case, the cell surface is contacted with a fluorescently labeled antibody. The cell/antibody complex is then subjected to a cell sorter, which is able to separate labeled from unlabeled cells (**Sekizawa et al, Fetal Diagn Ther 1999 Jul-Aug;14(4):229-33**). Alternatively, the cells may be isolated through affinity binding to an antibody which has previously been immobilized onto a solid support.

A person skilled in the art will recognize that different detection formats may be applied in order to perform the claimed genotype determination according to the invention. Most commonly in Real Time PCR, the amplification products are detected using fluorescent signals. This is possible by detecting the amplification products with a ds DNA binding fluorescent Dye such as Ethidium Bromide, SybrGreen or SybrGold (Molecular Probes).

Alternatively, fluorescently labeled hybridization probes may be used. Independent from the detection format or fluorescent label, Hybridization probes are always polynucleotides having sequences which are completely identical with or exactly complementary to the sequence of the target nucleic acid. Yet, it is also within the scope of the invention, if the probes contain one or several mismatches, as long as they are capable of hybridizing to the analyte under appropriate hybridization conditions. In any case, it has been proven to be particular advantageous, if the sequence identity or complementarity is 100% over a range of at least 10 contiguous residues. It has also been proven to be advantageous, if the length of the probe does not exceed 100 nucleotides, preferably not more than 40 nucleotides.

However, hybridization probes may have 5' or 3' overhangs which do not hybridize to the target nucleic acid.

The term "Polynucleotide" in this context summarizes not only (Desoxy)-Oligo-Ribonucleotides, but also all DNA- or RNA-derivatives known in the art like e.g. Methyl-Phosphonates, Phosphothioates , 2'-O-Alkyl-derivatives as well as Peptide Nucleic Acids, and analoga comprising modified bases like 7-Deaza-Purines.

Hybridization probes such as TaqMan or Molecular beacons may be used. Most preferred are FRET/Hybridization probes, i.e. a pair of adjacently hybridizing probes, wherein upon hybridization the two fluorescent moieties are brought into close vicinity such that Fluorescent Resonance Energy Transfer can take place. The term "FRET Hybridization probes" therefore is defined as a pair of hybridization probes, each probe carrying a fluorescent compound, which together may act as a FRET pair thus enabling the detection of a nucleic acid, when both probes are hybridized adjacently to a target molecule.

According to the invention, it is preferred, if the first and the second target sequence are amplified and detected in one tube, since quantification errors due to variable amounts of starting materials can be excluded. This is possible in a multiplex approach, wherein differentially labeled hybridization probes for each sequence are used for detection of the respective amplification products.

Such assays may be performed on a Light Cycler instrument (Roche Molecular Biochemicals) using a first pair of FRET Hybridization probes labeled with Fluorescein at the 3' end of the first oligonucleotide and with LC-Red-640 (Roche Molecular Biochemicals) at the 5' end of the second oligonucleotide and a second pair of FRET Hybridization probes labeled with Fluorescein at the 3' end of the first oligonucleotide and with LC-Red-705 (Roche Molecular Biochemicals) at the 5' end of the second oligonucleotide.

Principally, any kind of quantification method can be applied, however, it has been proven to be advantageous, if methods using an external standard are applied. The external standard itself may either be a plasmid or a linearized template with the target sequences to be amplified or, alternatively, genomic DNA wherein the phenotypes of the gene loci to become investigated.

In case of quantification of a nucleic acid using external standards, a calibration curve has to be generated. For this calibration curve, known amounts of the target nucleic acid are amplified and the intensity of fluorescent signal is determined as a function of cycle number. After smoothening of the kinetics by a mathematical fit, the first or second maximum of the derivative are calculated. This enables a correlation between the original target concentration and the fractional cycle number of a determined maximum. Subsequently, determination of unknown analyte concentrations may be performed.

In order to eliminate quantification errors originating from different detection sensitivities, it has been proven to be particular advantageous, if the same batch of hybridization probe(s) is used for the sample to be analyzed and for the calibration samples.

The claimed method can be used for analysis of multiple different gene loci as well as for the analysis of chromosomal abnormalities like whole chromosome aberrations, e.g the detection of trisomy disorders like trisomy 13, 18, or 21. The list of applications, however, is not restricted to the examples given below.

The reference gene always can be chosen almost arbitrarily, as long as it is possible to establish a quantitative and reproducible amplification reaction with respect to this target. Notwithstanding the foregoing, the reference allele should of course be reasonable stable and invariant within a given population. For analysis of whole chromosome aberrations, it is clear that the reference gene needs to be located on a chromosome different from the one with the allele to be investigated.

The genes encoding Superoxide Dismutase (SOD) located on Chromosome 21 and Myelin Basic protein (MBP) located on Chromosome 18 have been proven to be particular advantageous not only as reference genes but also as indicator alleles for detection of chromosomal abnormalities for trisomy 18 or trisomy 21. Even more preferred embodiments comprise the usage of one or more primers according to Seq. Id. No: 1-4, which may be detected by FRET-Hybridization probes according to Seq. Id. No. 5-6 for SOD and 7-8, respectively for MBP.

In another aspect, the invention is directed to the determination of the allelic status of RhD. This is preferably done on parental DNA, which is extremely important in case the mother is RhD negative. However, the assay can also be performed on fetal DNA. Appropriate primers are Oligonucleotides according to Seq. Id. No. 9-10. FRET-Hybridization probes according to Seq. Id. No. 11-12 may be used. SOD, MBP or any other target may be chosen as a reference gene.

Another aspect of the invention is directed to the determination of a female carrier status of a Chromosome X-linked genetic disorder like Heamophilia (Factor VIII) or Myopathy. For Heamophila, appropriate primers are Oligonucleotides according to Seq. Id. No. 13-14. FRET-Hybridization probes according to Seq. Id. No. 15-16 may be used. Again, SOD or MBP preferably may serve as reference genes.

Yet another aspect of the invention is directed to sex determination based on analysis of fetal DNA using the X-linked SRY gene and a second autosomal gene as reference alleles.

The following examples, references and sequence listing are provided to aid the understanding of the present invention, the true scope of which is set forth in the appended claims. It is understood that modifications can be made in the procedures set forth without departing from the spirit of the invention.

### Examples

### Harvest and culturing of amniotic fetal cells

Amniotic fetal cells are harvested from amniotic fluid by amniocentesis performed as soon as 14 weeks of pregnancy. The cells are obtained from 10ml of AF after centrifugation (5 min. at 1000rpm/min) and are processed in the following manner:
- cells are resuspended in 5ml of supernatant

1ml of this suspension is added to in a cell culture medium (ie HAM F10) and then incubated at 37°C with 5% CO2 in a incubator.

### Example 1: DNA sample preparation from fetal cells

For preparation of fetal genomic DNA from cultured cells, the HighPure PCR template preparation kit was used (Roche Molecular Biochemicals, Cat. No. 1 796 828) which is based on cell lysis by means of adding Guanidinium-HCL.

### Example 2: Real Time PCR amplification

LightCycler PCRs were set up in a final volume of 20 µl with the FastStart DNA Master Hybridization Probes Kit (Roche Molecular Biochemicals), each primer at a concentration of 0,5µM, each probe at a concentration of 0,25 µM and 5 µl of extracted DNA sample corresponding to about 10⁺³ cells, which had been isolated according to example 1. A hot-start procedure was systematically applied. Carryover contamination was prevented using heat-labile Uracil-DNA-Glycosylase (UNG, Roche Molecular Biochemicals). The reaction mix is listed in table 1 below:

**Table 1:**

| | Volume [µl] | [Final] |
|---|---|---|
| LC Fast-DNA Master Hybridization Probes | 2 | 1x |
| (including FastStartTaq-Polymerase) | | |
| MgCl₂ (25mM) | 3.2 | 5mM |
| Primers (20µM each) Target Gene | 0.5 | 0.5µM |
| Hybridization probes LCRed 640 (20µM each) | 0.25 | 0.25µM |
| Primers (20µM each) Reference Gene | 0.5 | 0.5µM |
| Hybridization probes LCRed705 (20µM each) | 0.25 | 0.25µM |
| Uracil-DNA glycosylase (1U/µl) | 0.25 | 0.25 U |
| Standard DNA in appropriate dilution | 5 | |
| H₂O (PCR grade) | 8,05 | |
| Total volume | 20 | |

For unknown samples, replace the standard DNA with sample DNA extract.

The reaction mixture was initially incubated for 1min at room temperature to allow UNG to act. This incubation was followed by a **8-min step at 95°C** to denature the DNA, to inactivate UNG, and **to activate Taq DNA polymerase**. Amplification was performed in a LightCycler (Roche Molecular Biochemicals) according to the following temperature cycling protocol:

| | |
|---|---|
| Denaturation | 95°C for 10s (ramp rate 20°C/s) |
| | |
| Annealing | 60°C for 10s (ramp rate 20°C/s) |
| | |
| Extension | 72°C for 15s (ramp rate 2°C/s) |

During thermocycling, a single fluorescence reading for each sample was taken at the annealing step. Quantitative results were expressed by determination of the crossing point Cp which marked the cycle when the measured fluorescent signal exceeded a certain threshold of signal intensity. Subsequently, the Cp's were plotted against an external standard calibration curve of known concentrations of the target nucleic acid, which had been amplified and measured previously. Genomic DNA with known phenotype always served as an external standard.

### Example 3: Detection of trisomy 21 and 18

Using the SOD gene as a marker for Chromosome 21 and the MBP gene as a marker for Chromosome 18, an analysis for trisomy 18 and 21 was performed such that trisomy 21 was expected to result in an increased gene dosage of SOD and trisomy 18 was expected to result in an increased dosage of MBP.

Sample preparation was performed according to example 2. Real time PCR was carried out according to example 2.

For amplification and detection of SOD, primers according to Seq. Id. No: 1 and 2 and FRET-Hybridization probes according to Seq. Id. No. 5 (labeled with Fluorescein at its 3' end) and Seq. Id. No: 6 (labeled with LC-Red-640 at its 5' end) were used. For amplification and detection of MBP, primers according to Seq. Id. No: 3 and 4 and FRET-Hybridization probes according to Seq. Id. No. 7 (labeled with Fluorescein at its 3' end) and Seq. Id. No: 8 (labeled with LC-Red-705 at its 5' end) were used. Amplification of both targets was carried out in a single tube, however, each sample was amplified and measured at least in duplicate. All results with respect to the determined Genotype were confirmed by conventional Caryotype analysis using conventional techniques of chromosomal spreading and light microscopy. The obtained data from 2 independent experiments resulting in different detection sensitivities are listed in Table 2.

**Table 2**

| Sample No. | SOD ng/µl | MBP ng/µl | Ratio SOD : MBP | Caryotype |
|---|---|---|---|---|
| 1 | 204 | 97 | 2.11 | 46 + Chr 21 |
| 2 | 1235 | 862 | 1.43 | 46 + Chr 21 |
| 3 | 726 | 985 | 0.74 | 46 |
| 4 | 21 | 64 | 0.32 | 46 + Chr 18 |
| 5 | 1177 | 853 | 1.38 | 46 + Chr 21 |
| 6 | 1412 | 1767 | 0.80 | 46 |
| 7 | 10.67 | 7.27 | 1.47 | 46 + Chr 21 |
| 8 | 1.95 | 2.60 | 0.75 | 46 + Chr 18 |
| 9 | 3.45 | 3.95 | 0.87 | 46 |
| 10 | 15.42 | 11.79 | 1.31 | 46 + Chr 21 |
| 11 | 1.39 | 1.28 | 1.09 | 46 |
| 12 | 9.45 | 8.45 | 1.21 | 46 + Chr 21 |
| 13 | 3.46 | 3.80 | 0.91 | 46 |

The data of this table 2 are from 2 independent experiments with different batches of hybridization probes; samples 1-6 were assayed in the first experiment, and samples 7-13 were assayed in the second experiment. As shown, a normal genotype resulted in a SOD/MBP ratio between 0, 74 and 0,80 in the first experiment and a ratio between 0,87 and 1,09 in the second experiment. A trisomy 21 genotype resulted in a SOD/MBP ratio of at least 1.21 for both experiments. A trisomy 18 genotype resulted in a SOD/MBP ratio of about 0.32 in the first experiment and a ratio of 0.75 in the second experiment.

These data clearly demonstrate that the disclosed method is applicable for diagnosis of trisomies, as soon as enough material from the individual to be analyzed is available in order to perform a typical Real Time PCR experiment, provided, however, that intra assay variances are excluded.

### Example 4: Determination of RhD genotype

DNA from 101 individuals was basically analyzed as disclosed in example 3. For amplification and detection of RhD, primers according to Seq. Id. No: 9 and 10 and FRET-Hybridization probes according to Seq. Id. No. 11 (labeled with Fluorescein at its 3' end) and Seq. Id. No: 12 (labeled with LC-Red-640 at its 5' end) were used. MBP was detected as a reference gene in the same samples using primers and FRET-Hybridization probes according to example 3.

For this approach, the RhD/MBP ratio was expected to be increased in case of a homozygous DD genotype as compared to a heterozygous Dd genotype, whereas in case of a homozygous dd genotpype resulting from a complete deletion of the RhD gene, amplification of RhD does not occur. All results with respect to the determined Genotype/Phenotype were confirmed by a conventional RhD agglutination assay (**Diamed**). The obtained data are listed in table 3:

**Table 3**

| **Sample No.** | **RhD** **ng/µl** | **MBP** **ng/µl** | **Ratio** **MBP/RhD** | **Phenotype in agglutination assay** |
|---|---|---|---|---|
| 1 | 4.1 | 4.7 | 1.15 | DD |
| 2 | 18.5 | 21.3 | 1.15 | DD |
| 3 | 17.3 | 20.1 | 1.16 | DD |
| 4 | 8.9 | 10.4 | 1.17 | DD |
| 5 | 21.5 | 25.5 | 1.19 | DD |
| 6 | 24.2 | 28.9 | 1.19 | DD |
| 7 | 12.3 | 14.7 | 1.20 | DD |
| 8 | 17.1 | 20.7 | 1.21 | DD |
| 9 | 47.8 | 58.1 | 1.22 | DD |
| 10 | 6 | 7.3 | 1.22 | DD |
| 11 | 31.2 | 38.0 | 1.22 | DD |
| 12 | 22 | 27 | 1.23 | DD |
| 13 | 23.7 | 29.1 | 1.23 | DD |
| 14 | 21 | 25.8 | 1.23 | DD |
| 15 | 22.6 | 27.8 | 1.23 | DD |
| 16 | 15 | 18.5 | 1.23 | DD |
| 17 | 22.5 | 27.8 | 1.24 | DD |
| 18 | 36.5 | 45.2 | 1.24 | DD |
| 19 | 17.6 | 22 | 1.25 | DD |
| 20 | 30.2 | 37.9 | 1.25 | DD |
| 21 | 15.9 | 20 | 1.26 | DD |
| 22 | 22.4 | 28.3 | 1.26 | DD |
| 23 | 38.6 | 49 | 1.27 | DD |
| 24 | 18.1 | 23 | 1.27 | DD |
| 25 | 22.1 | 28.1 | 1.27 | DD |
| 26 | 20.6 | 26.2 | 1.27 | DD |
| 27 | 45.7 | 58.2 | 1.27 | DD |
| 28 | 45.1 | 57.5 | 1.27 | DD |
| 29 | 30.1 | 38.4 | 1.28 | DD |
| 30 | 12.3 | 15.7 | 1.28 | DD |
| 31 | 13.2 | 16.9 | 1.28 | DD |
| 32 | 23.9 | 30.6 | 1.28 | DD |
| 33 | 10.4 | 13.4 | 1.29 | DD |
| 34 | 20.6 | 26.8 | 1.30 | DD |
| 35 | 28.5 | 37.7 | 1.32 | DD |
| 36 | 20.1 | 26.6 | 1.32 | DD |
| 37 | 8.9 | 11.8 | 1.33 | DD |
| 38 | 29.2 | 39 | 1.34 | DD |
| 39 | 14.7 | 19.7 | 1.34 | DD |
| 40 | 18.3 | 24.8 | 1.36 | DD |
| 41 | 14.2 | 19.4 | 1.37 | DD |
| 42 | 22.6 | 31.5 | 1.39 | DD |
| 43 | 12.1 | 17 | 1.40 | DD |
| 44 | 14 | 19.9 | 1.42 | DD |
| 45 | 18.9 | 27 | 1.43 | DD |
| 46 | 14.6 | 22.2 | 1.52 | DD |
| 47 | 53 | 80.6 | 1.52 | DD |
| 48 | 11.4 | 25.2 | 2.21 | Dd |
| 49 | 8.5 | 20 | 2.35 | Dd |
| 50 | 16.8 | 39.6 | 2.36 | Dd |
| 51 | 11.2 | 26.5 | 2.37 | Dd |
| 52 | 9.7 | 23.1 | 2.38 | Dd |
| 53 | 5.9 | 14.1 | 2.39 | Dd |
| 54 | 15.8 | 37.9 | 2.40 | Dd |
| 55 | 17.3 | 41.5 | 2.40 | Dd |
| 56 | 3.5 | 8.4 | 2.40 | Dd |
| 57 | 16.9 | 40.6 | 2.40 | Dd |
| 58 | 10.2 | 24.4 | 2.40 | Dd |
| 59 | 7.5 | 18.1 | 2.41 | Dd |
| 60 | 2.9 | 7 | 2.41 | Dd |
| 61 | 11.3 | 27.4 | 2.42 | Dd |
| 62 | 10.8 | 26.2 | 2.43 | Dd |
| 63 | 12.6 | 32.1 | 2.55 | Dd |
| 64 | 10.5 | 26.8 | 2.55 | Dd |
| 65 | 17.6 | 45 | 2.56 | Dd |
| 66 | 11 | 28.2 | 2.56 | Dd |
| 67 | 32.7 | 84.1 | 2.57 | Dd |
| 68 | 9.4 | 24.2 | 2.57 | Dd |
| 69 | 15.3 | 39.5 | 2.58 | Dd |
| 70 | 8.4 | 21.7 | 2.58 | Dd |
| 71 | 13.6 | 35.2 | 2.59 | Dd |
| 72 | 6.1 | 15.8 | 2.59 | Dd |
| 73 | 8.5 | 22.1 | 2.60 | Dd |
| 74 | 12.7 | 33.1 | 2.61 | Dd |
| 75 | 22.1 | 57.6 | 2.61 | Dd |
| 76 | 7.3 | 19.1 | 2.62 | Dd |
| 77 | 7.2 | 18.9 | 2.63 | Dd |
| 78 | 3.5 | 9.2 | 2.63 | Dd |
| 79 | 12.3 | 32.5 | 2.64 | Dd |
| 80 | 20.0 | 54.0 | 2.70 | Dd |
| 81 | 16.8 | 45.4 | 2.70 | Dd |
| 82 | 24.5 | 67.1 | 2.74 | Dd |
| 83 | 12.3 | 33.7 | 2.74 | Dd |
| 84 | 5.1 | 14 | 2.75 | Dd |
| 85 | 24.7 | 67.9 | 2.75 | Dd |
| 86 | 11.2 | 30.9 | 2.76 | Dd |
| 87 | 6.4 | 17.8 | 2.78 | Dd |
| 88 | 6.8 | 19.3 | 2.84 | Dd |
| 89 | 26.2 | 74.4 | 2.84 | Dd |
| 90 | 6.5 | 18.6 | 2.86 | Dd |
| 91 | 10.6 | 31.1 | 2.93 | Dd |
| 92 | NEG | ok | | Dd |
| 93 | NEG | OK | | Dd |
| 94 | NEG | ok | | Dd |
| 95 | NEG | ok | | Dd |
| 96 | NEG | Ok | | Dd |
| 97 | NEG | ok | | Dd |
| 98 | NEG | ok | | Dd |
| 99 | NEG | ok | | Dd |
| 100 | NEG | Ok | | Dd |
| 101 | NEG | Ok | | Dd |

As shown in Table 3, a DD genotype resulted in a MBP/RhD ratio of about 2,57 with a standard deviation of +/- 0,17. A Dd genotype resulted in a MBP/RhD ratio of about 1,28 with a standard deviation of about +/-0,09, whereas amplification could not be observered in case of the dd genotype. These data clearly show that using this method, the RhD genotype can be determined reliably.

### Example 5: Determination of Haemophilia carrier status

DNAs from 31 individuals were analyzed basically according to example 3. Due to occuring Haemophilia in male member of their family (No. 26), 3 females (No. 29,30, and 31) were suspected to have a heterozygous Factor VIII molecular defect resulting in a Haemophilia carrier status.

Analysis was principally performed as disclosed in example 4. For amplification and detection of Factor VIII, exon 1, primers according to Seq. Id. No: 13 and 14 and FRET-Hybridization probes according to Seq. Id. No. 15 (labeled with Fluorescein at its 3' end) and Seq. Id. No: 16 (labeled with LC-Red-705 at its 5' end) were used. SOD was detected as a reference gene in the same samples using primers and FRET-Hybridization probes according to example 3.

For this approach, the FactorVIII/SOD ratio in female carriers was expected to be different to the ratio observed in wild type female individuals but similar to male individuals, since both male individuals and female carriers carry one intact copy of the Factor VIII gene. The obtained data are listed in table 4. The table shows the values of two independendent experiments using different batches of hybridizazion probes. The different experiments are indicated by sample numbers 1-25 and 26-31.

**Table 4:**

| Sample No. | Factor VIII Ng/µl | SOD ng/µl | Ratio SOD: Factor VIII | SEX Carrier (c) |
|---|---|---|---|---|
| 1 | 24.8 | 24.7 | 1.00 | XX |
| 2 | 27.7 | 28.1 | 1.01 | XX |
| 3 | 17.7 | 18.7 | 1.06 | XX |
| 4 | **13.7** | **26.4** | **1.93** | **XY** |
| 5 | 65.8 | 74.3 | 1.13 | XX |
| 6 | **8.6** | **18.0** | **2.09** | **XY** |
| 7 | 39.1 | 40.5 | 1.04 | XX |
| 8 | 27.1 | 28.5 | 1.05 | XX |
| 9 | 38.2 | 41.2 | 1.08 | XX |
| 10 | **14.3** | **28.9** | **2.02** | **XY** |
| 11 | 23.9 | 22.7 | 0.95 | XX |
| 12 | **7.9** | **15.6** | **1.97** | **XY** |
| 13 | 72.3 | 76.5 | 1.06 | XX |
| 14 | 46.1 | 48.9 | 1.06 | XX |
| 15 | **23.9** | **48.3** | **2.02** | **XY** |
| 16 | **22.1** | **43.9** | **1.99** | **XY** |
| 17 | 34.8 | 39.1 | 1.12 | XX |
| 18 | **11.7** | **22.2** | **1.90** | **XY** |
| 19 | **19.2** | **38.3** | **1.99** | **XY** |
| 20 | 37.4 | 38.5 | 1.03 | XX |
| 21 | 47.2 | 43.4 | 0.92 | XX |
| 22 | **11** | **20.8** | **1.89** | **XY** |
| 23 | **13.5** | **26.4** | **1.96** | **XY** |
| 24 | 37.2 | 40.2 | 1.08 | XX |
| 25 | 30.2 | 30.9 | 1.02 | XX |
| 26 | Neg/Del | 15.9 | #VALUE! | **XY,** Haemophillic |
| 27 | 6.9 | 15.5 | 2.25 | XX |
| 28 | 21 | 52.3 | 2.49 | XX |
| 29 | 19.4 | 15.5 | 0.80 | XX, Carrier |
| 30 | 18.8 | 16.2 | 0.86 | XX, Carrier |
| 31 | 14.2 | 11.5 | 0.81 | XX, carrier |

As it can be deduced from the table, in each of the two independent experiments, two clear non overlapping populations can be identified: a) values found for female individuals definitely carrying two alleles of Factor VIII, and b) values for male individuals carrying one allele. The ratios obtained for the two female suspected to be haemophilia carrriers were indeed similar to the values obtained for male individuals suggesting that they are Haemophilia carriers.

### List of references

Bernard, et al., Analytical Biochemistry 235, p. 101-107 (1998)
Lizardi et al., US 5, 118,801
Lo et al, Lancet. 1997 Aug 16;350(9076):485-7
Lo et al., New England J. of Medicine 339, p. 1734-1738, 1998
Pertl et al, Semin Perinatol 1999 Oct;23(5):393-402
Philip et al, Prenat Diagn 1994 Dec;14(13):1203-15
Roche Molecular Biochemicals, Cat. No. 1 796 828
Samura et al, Prenat Diagn 2000 Apr;20(4):281-6
Sekizawa et al, Fetal Diagn Ther 1999 Jul-Aug;14(4):229-33
UNG, Roche Molecular Biochemicals
U.S. 4,683,102 (Mullis et al.)
U.S. 4,683,195
Wang et al, Cytometry 2000 Mar 1;39(3):224-30
Whittle, Arch Dis Child 1992 Jan;67(1 Spec No):65-8
Wittwer et al., Biotechniques, Vol. 22, No, 1, 130-138, 1997
WO 97/46707
WO 97/46712
WO 97/46714 (Wittwer et al.)

### Annex to the application documents - subsequently filed sequences listing

SEQUENCE LISTING

<110> Roche Diagnostics GmbH

<120> New Method for Genotype Determination

<130> 5383/00/EP

<140> EP01102106.0
<141> 2001-01-31

<160> 16

<170> PatentIn Ver. 2.1

<210> 1
<211> 20
<212> DNA
<213> Artificial Sequence

<220>
<223> Description of Artificial Sequence:primer

<400> 1
caccacccag gcatcattag
20

<210> 2
<211> 23
<212> DNA
<213> Artificial Sequence

<220>
<223> Description of Artificial Sequence:primer

<400> 2
agttcggcag atttcagttc att
23

<210> 3
<211> 21
<212> DNA
<213> Artificial Sequence

<220>
<223> Description of Artificial Sequence:primer

<400> 3
ccacttgccc ctgttaccta a

21

<210> 4
<211> 22
<212> DNA
<213> Artificial Sequence

<220>
<223> Description of Artificial Sequence:primer

<400> 4
gttctcattt aactgttggc cg
22

<210> 5
<211> 23
<212> DNA
<213> Artificial Sequence

<220>
<223> Description of Artificial Sequence:primer

<400> 5
ggcggatccc ttggcaagtt tac
23

<210> 6
<211> 24
<212> DNA
<213> Artificial Sequence

<220>
<223> Description of Artificial Sequence:primer

<400> 6
cccaaagcag ctctctcgtg tctg
24

<210> 7
<211> 23
<212> DNA
<213> Artificial Sequence

<220>
<223> Description of Artificial Sequence:primer

<400> 7
ccgtagtagg gtcaggccac ggc

23

<210> 8
<211> 23
<212> DNA
<213> Artificial Sequence

<220>
<223> Description of Artificial Sequence:primer

<400> 8
ccctaatgcc tgcggagttg tgc
23

<210> 9
<211> 20
<212> DNA
<213> Artificial Sequence

<220>
<223> Description of Artificial Sequence:primer

<400> 9
gcctgcattt gtacgtgaga
20

<210> 10
<211> 21
<212> DNA
<213> Artificial Sequence

<220>
<223> Description of Artificial Sequence:primer

<400> 10
caaagagtgg cagagaaagg a
21

<210> 11
<211> 23
<212> DNA
<213> Artificial Sequence

<220>
<223> Description of Artificial Sequence:primer

<400> 11
gcaggcactg gagtcagaga aaa

23

<210> 12
<211> 24
<212> DNA
<213> Artificial Sequence

<220>
<223> Description of Artificial Sequence:primer

<400> 12
tgacagcaaa gtctccaatg ttcg
24

<210> 13
<211> 22
<212> DNA
<213> Artificial Sequence

<220>
<223> Description of Artificial Sequence:primer

<400> 13
tcctccaagc agacttacat cc
22

<210> 14
<211> 20
<212> DNA
<213> Artificial Sequence

<220>
<223> Description of Artificial Sequence:primer

<400> 14
tgggtgcagt ggaactgtca
20

<210> 15
<211> 24
<212> DNA
<213> Artificial Sequence

<220>
<223> Description of Artificial Sequence:primer

<400> 15
caggcagctc accgagatca cttt

24

<210> 16
<211> 23
<212> DNA
<213> Artificial Sequence

<220>
<223> Description of Artificial Sequence:primer

<400> 16
ggacatggct ttaccttgcg tcc
23

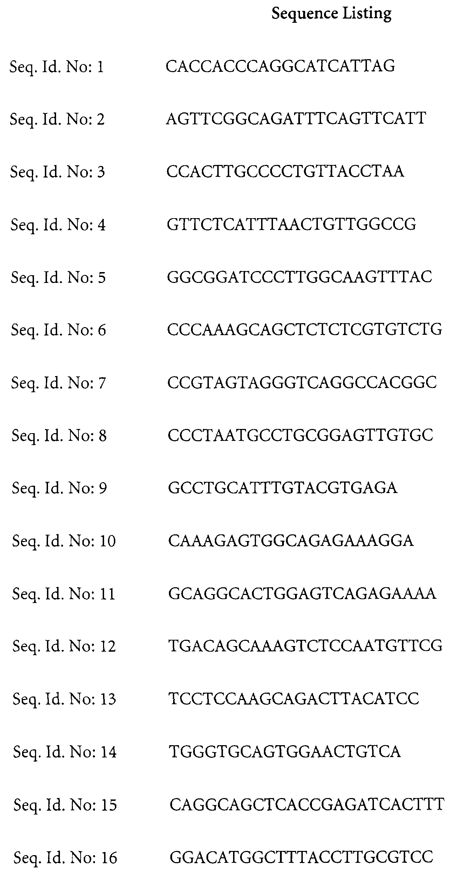

## Claims

1. Method for genotype determination at a specific gene locus of an individual or a fetus comprising
a) Amplifying a first sequence of said gene locus and a second sequence of a second reference gene locus from DNA originating from a sample containing biological material of said individual or fetus
b) Quantifying the original amount of DNA of said first gene locus and said second gene locus present in the sample
c) Calculating the ratio between the amount of DNA from the first gene locus and the amount of DNA from the second gene locus originally present in the sample prior to the amplification reaction

2. Method for genotype determination at a specific gene locus of an individual or a fetus comprising
a) Amplifying a first sequence of said gene locus and a second sequence of a second reference gene locus from DNA originating from a sample containing biological material of said individual or fetus
b) Monitoring both amplifications in real time and determining the amount of amplification products after each cycle
c) Calculating the ratio between the amount of DNA from the first gene locus and the amount of DNA from the second gene locus

3. Method for genotype determination at a specific gene locus of a fetus comprising
a) Isolating fetal cells, preferably by Immuno-enrichment
b) Amplifying a first sequence of said gene locus and a second sequence of a second reference gene locus from DNA originating from cells isolated in step a)
c) Monitoring both amplifications in real time and determining the amount of amplification products after each cycle
d) Calculating the ratio between the amount of DNA from the first gene locus and the amount of DNA from the second gene locus originally present in the sample prior to the amplification reaction method

4. Method according to claim 1-3 , wherein said first and said second sequence are amplified and detected in one tube

5. Method according to claim 1-4, wherein the amplification products are detected using fluorescent signals

6. Method according to claim 5, wherein the amplification probes are detected with FRET/Hybridization probes

7. Method according to claims 1-6, wherein either said specific gene locus or said reference gene locus is MBP or SOD.

8. Use of a method according to claims 1-7, wherein either said specific gene locus or said reference gene locus is the RhD gene.

9. Use of a method according to claims 1-7 for detecting chromosomal abnormalities

10. Use according to claim 9 for determination of trisomy 21, trisomy 18, or trisomy 13

11. Use according to claim 9 for determination of microdeletional syndromes

12. Use according to claim 9 for determination of a female carrier status for a Chromosome X-linked genetic disorder

13. Use according to claim 9, wherein said genetic disorder is Heamophilia or Myopathy

14. Use of a method according to claims 1-7, for sex determination
